(19) Europäisches Patentamt
European Patent Office
Office européen des brevets

(11) EP 4 553 560 A1

(12) EUROPEAN PATENT APPLICATION

(43) Date of publication:
14.05.2025 Bulletin 2025/20

(21) Application number: 24208616.3

(22) Date of filing: 24.10.2024

(51) International Patent Classification (IPC):
G02B 23/24 (2006.01)     G02B 27/10 (2006.01)
G02B 27/14 (2006.01)

(52) Cooperative Patent Classification (CPC):
A61B 1/051; A61B 1/00096; A61B 1/00186;
A61B 1/043; A61B 1/0638; G02B 13/16;
G02B 23/04; G02B 23/24; G02B 23/243;
G02B 27/1013; G02B 27/14; G02B 27/141

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.10.2023  DE 102023130062

(71) Applicant: Karl Storz SE & Co. KG
78532 Tuttlingen (DE)

(72) Inventors:
• BARTH, Sebastian
  78532 Tuttlingen (DE)
• BAUER, Franz
  78606 Seitingen-Oberflacht (DE)

(54) **IMAGING DEVICE FOR A DISTAL END SECTION OF AN ENDOSCOPE, OBJECTIVE SYSTEM AND ENDOSCOPE**

(57) The present invention relates to an imaging device for a distal end section of an endoscope, wherein the imaging device comprises a pentaprism with a main body and a prism wedge, and at least a first image sensor for capturing light of a first spectral region and a second image sensor for capturing light of a second spectral region are assignable to the imaging device, and a dichroic beam splitting layer as a first internal reflective and transmissive splitting layer is arranged between the main body and the prism wedge of the pentaprism, so that incident beams comprising the first and second spectral regions are split by the dichroic beam splitting layer into first beams of the first spectral region and second beams of the second spectral region, wherein at least one separate optical element is arranged between the pentaprism and the first image sensor and/or the second image sensor for changing a property of the light of the first spectral region and/or the second spectral region. Furthermore, the invention concerns an objective system and an endoscope.

Fig. 3

EP 4 553 560 A1

**Description**

The present invention relates to an imaging device for a distal end section of an endoscope. Furthermore, the invention concerns an objective system and an endoscope comprising such an imaging device.

Particularly, the invention refers to an imaging device comprising a pentaprism with a main body and a prism wedge and an optical axis and a dichroic beam splitting layer as a first internal reflective and transmissive splitting layer, wherein an entrance surface, a second internal reflective surface and a first exit surface are arranged on the main body and a second exit surface is arranged on the prism wedge, and at least a first image sensor for capturing light of a first spectral region and a second image sensor for capturing light of a second spectral region are assignable to the imaging device, wherein the first spectral region and the second spectral region differ at least partially from each other, and the dichroic beam splitting layer is arranged between the main body and the prism wedge of the pentaprism, so that incident beams comprising the first and second spectral regions are split by the dichroic beam splitting layer into first beams of the first spectral region and second beams of the second spectral region.

Endoscopes for use in medical and/or non-medical applications may utilize both white light imaging and fluorescence imaging. Endoscopic instruments intended for industrial use, rather than medical use, are often referred to as borescopes. As this invention relates to both medical endoscopes and borescopes the term "endoscope" is used to generally include both instruments. Conventional endoscopes able to capture both white light and fluorescence images with a single optical path and only a single image sensor do so by collecting alternating frames with staggered white light and excitation illumination. Such system arrangement results in a frame rate that is significantly lower than a frame rate of a system using only visible white light, typically reaching, at most, half of the frame rate of a white light only system. The low frame rate leads to unwanted motion blurring. In addition to the necessary shuttering between white light and fluorescence frames, the sensitivity of the fluorescence frames is limited by the shared optical path, which typically results in the inability to attenuate one signal relative to the other signal. Image processing subsequently analyzes and combines the images from alternating frames into a fluorescence/white light overlay with a low overall frame rate. This technique results in a lower sensitivity in the fluorescence range as well as a reduced brightness and resolution of the overlaid image compared to a white light only image.

An alternative to using only one image sensor, shuttering the frames, and overlaying the white light and fluorescence images, would be to place two complete objective systems in an endoscope for capturing white light and fluorescence images separately. However, due to the space requirements of two objective systems in parallel, not to mention the added complexity of orientation, increased expense, and loss of image brightness, this is not a feasible solution for chip-on-the-tip (COTT) endoscopes, where the respective electronic image sensor or sensors is/are arranged in the distal end section, e.g., the tip of the narrow shaft of the endoscope.

Instead of using two complete objective systems, it is also possible to use just one objective system and split the beams afterwards by means of a beam splitter for directing the split beams onto two different image sensors. However, it is not possible to miniaturize the conventional beam splitter and the two image sensors into the small space provided by COTT endoscopes. For example, splitting an afocal beam path including both white and fluorescence light by means of a 45° beam splitter has the disadvantage, besides providing the necessary afocal beam path itself, that a subsequent focusing is required. Otherwise, when using a conventional beam splitter in the converging beam path in front of the image sensors, aberrations occur on the sensor that result in transmission and the image quality decreasing. Overall, this results in large imaging devices due to the necessary refocusing.

US 2020/0088579 A1 discloses a hybrid spectral imager comprising multiband filtering optics with a beam divider means for generating at least two replica images of a target image and a tunable multiband filtering means interposed into the imaging path and effecting a tunable multiband pass filtering in the image replicas. The beam divider can be a pentaprism, wherein one of its five surfaces is coated with a polychroic mirror substrate and this coated surface is cemented together with a triangle prism for making the prism's rear surface parallel to the front surface of the pentaprism. Furthermore, the pentaprism comprises a tilting actuator to adjust the tilting angle of the prism assembly and therewith the direction of the imaging ray beams. The images of a target object are focused by an objective lens optics and divided by the tilting prism assembly onto two image sensors, wherein, at each tilting step, a new set of images is obtained simultaneously in a snapshot mode, so that, in a scanning mode, the entire spectrum can be scanned to create a complete spectral cube for the target object. Hereby, for adapting to a variety of sizes of sensor arrays, the path lengths of the two split beams have to be substantially equal. Consequently, the arrangement of this hybrid spectral imager in a narrow shaft of a COTT endoscope is limited.

In US 2014/0225992 A1 a minimally invasive surgical system with an image capture unit including a prism assembly and a sensor assembly is described. The image capture unit includes a shared lens assembly followed by a sensor assembly, wherein the sensor assembly includes a prism assembly, a reflective unit and co-planar image capture sensors. Hereby, the two image capture sensors having similar image sizes are symmetric across a plane that intersects the longitudinal axis of the stereoscopic endoscope and only a small gap is arranged between the respective surface of the prism assembly and each co-planar image capture sensor.

The objective of the present invention is to improve the known state of the art.

The problem is solved by an imaging device for a distal end section of an endoscope, wherein the imaging device comprises a pentaprism with a main body and a prism wedge and an optical axis and a dichroic beam splitting layer as a first internal reflective and transmissive splitting layer, and an entrance surface, a second internal reflective surface and a first exit surface are arranged on the main body and a second exit surface is arranged on the prism wedge, and at least a first image sensor for capturing light of a first spectral region and a second image sensor for capturing light of a second spectral region are assignable to the imaging device, wherein the first spectral region and the second spectral region differ at least partially from each other, and the dichroic beam splitting layer is arranged between the main body and the prism wedge of the pentaprism, so that incident beams comprising the first and second spectral regions are split by the dichroic beam splitting layer into first beams of the first spectral region and second beams of the second spectral region, wherein at least one separate optical element is arranged between the pentaprism and the first image sensor and/or the second image sensor for changing a property of the light of the first spectral region and/or the second spectral region, so that two separate images are capturable by the first image sensor and the second image sensor for superimposed imaging of the light of the first spectral region and the second spectral region. The two separate images are optimized for superimposed imaging of a target object using the light of the first spectral region and the second spectral region.

Therewith, an imaging device is provided for a chip-on-the-tip endoscope enabling the optimized simultaneous capturing of images of two different spectral regions by two separate image sensors meeting the restricted space available in the shaft of this kind of endoscope. Consequently, an increase of the overall frame rate and an optimized image quality can be achieved in a small, limited space in a shaft of a video endoscope. Due to the specific design of the pentaprism and the at least one separate optical element arranged between the pentaprism and the respective image sensor or sensors, the first and second split beams can be further optimized and/or corrected independently from each other for superimposed imaging of the light of both spectral regions at the best available imaging quality. Therewith, at least two optical well-corrected, separate images can be captured in a miniaturized design of the imaging device that can be or is arranged into or in the small space available in shafts of COTT endoscopes.

Due to the specific pentaprism design, an afocal beam bundle does not have to be provided by an objective lens system upstream of the imaging device, wherein an afocal beam bundle cannot be realized due to construction and space requirements in the narrow shaft of COTT endoscopes. Instead, the pentaprism can be arranged in a converging beam path and is designed such that both image sensors receive simultaneously the image from the object space without aberrations.

Furthermore, due to the specific geometric and space-saving design of the pentaprism, a further optimization of the relative beam path is enabled by the at least one separate optical element arranged between the pentaprism and the respective sensor, wherein the respective separate optical element can be present only in one of both respective paths or similar or different optical elements can be arranged in both respective paths. Therewith, the sensitivity, image brightness and/or resolution of a defined spectral region can be optimized without necessarily influencing the optical parameters of the other spectral region to be captured by the respective image sensor.

One of the primary innovations of the invention is the special design of the pentaprism with a prism wedge in combination with a downstream at least one separate optical element arranged between the pentaprism and at least one of the respective two image sensors for providing the simultaneous capturing of two images at a high frame rate and a high sensitivity and enabling the imaging device to be arranged in the small, limited space of a shaft of a COTT endoscope. Therewith, both image sensors can be used with full frame rate and optimized and/or specially adapted image size and/or properties. Therewith, a space-saving geometry of the imaging device due to the special design of the pentaprism in combination with the downstream arranged at least one separate optical element is realized which allows an easy fitting of the imaging device and/or the assignable image sensors inside the shaft of an endoscope in the longitudinal and radial direction of the shaft and enabling a simultaneously optimized capturing of separate images in the first spectral region and the second spectral region, each by a separate, dedicated image sensor, resulting in adapted and/or improved image quality. Due to the design of the pentaprism in combination with the separate optical element, a frame rate of 60 frames per second or even more can be achieved.

As utilized in accordance with the present disclosure, the following terms, unless otherwise indicated, shall be understood to have the following meanings.

An "endoscope", in particular a video endoscope, is an endoscope with a means for digital image acquisition in and/or at the distal end of an elongate shaft, and the transmission of data therefrom, for example, to the proximal end of the endoscope. The endoscope comprises the elongate shaft and a handle which are connectable to each other. In the present invention, at least two digital image sensors are located in the elongate shaft or at the distal end of the elongate shaft for image acquisition. In particular, the video endoscope is any kind of digital endoscope, for example a 2D colonoscope, a laparoscope or gastroenteroscope or a 3D video endoscope. In particular, the endoscope is a chip-on-the-tip endoscope (COTT).

The "elongate shaft" is, in particular, a rigid, semi-flexible or flexible tube. In particular, the shaft is configured for being inserted into a cavity to be viewed endoscopically, for example a cavity of a human or animal body. In industrial applications, the endoscope, or borescope, shaft will be placed into an element such as a pipe or another area which is difficult to access directly, such as behind a wall. Generally, the shaft may have an outer diameter in the range of 4 mm to 10 mm. Besides the objective system, the imaging device and two or more image sensors, the shaft may comprise one or more channels for irrigation or passing through working instruments (generally referred to as "working channels") in order to achieve the desired effect in the cavity or opening. The shaft can be detachably connected at its proximal end to a handle or be permanently connected thereto. The distal end section of the elongate shaft is the section remote from the user, while the proximal end section of the shaft is closer to the user.

An "objective system" is an optical system which includes an objective lens system to receive, pass forward and/or modify the image light from a target object space, wherein a beam of the image light passes through the objective system.

An "objective lens system" comprises in particular, in an order from an object side, a cover glass and/or a first lens, a second lens, and optional further lenses, which are arranged along an optical axis of the lens system. Advantageously, the optical axis can correspond to the optical axis of the pentaprism. Optionally, one or more optical filters can be located between any two lenses of the lens system.

A "lens" is, in particular, a transmissive optical body that focusses or disperses light beams (light rays) by means of refraction. The first lens, the second lens and optional further lenses can be single lenses, which are separated by an air gap, or are in contact to adjacent lenses at most pointwise. Also, a lens can be a combined, compound, lens and/or a rod lens. Preferably, the lenses are made of glass and/or a crystalline material.

An "imaging device" is, in particular, a device which further modifies the image light passed forward by the objective system and divides the image light to the at least two image sensors. The imaging device at least comprises the pentaprism, the at least one separate optical element and at least two image sensors for capturing the image are assignable or are included. The imaging device does not necessarily have to be arranged in the distal end section of the shaft close to the objective system, but can be located in a distance to the objective system in the distal end section.

A "pentaprism" is an optical component in form of a geometric body prism with five sides. The pentaprism in particular comprises a main body and a prism wedge together forming the five reflecting, transmitting and/or non-beam passing sides of the pentaprism in a longitudinal section view. The pentaprism can be used for different optical effects. The optical properties of the pentaprism in particular depend on the angles and/or the position of the optically effective prism surface in relation to one another and on the refractive indexes of the material of the main body and the prism wedge. In particular, the main body and the prism wedge comprise glass, and preferably the glass of the main body and the prism wedge are selected to have the same, or nearly the same, refractive index. For example, the refractive indexes of the main body and the prism wedge can be both 1.6. In a sectional view parallel to the travel direction of the incident light and therewith in a longitudinal section, the pentaprism has a substantial pentagon shape. In particular, the pentaprism has a length in the longitudinal direction and therewith along the optical axis of less than 5.0 mm. In particular, the pentaprism has a diameter of less than 3.5 mm perpendicular to the optical axis. The prism wedge is preferably arranged proximal of the main body and cemented to the main body. The cemented prism wedge in particular prevents a beam shift error on the second image sensor, arranged in particular in the proximal direction perpendicular to the optical axis.

An "entrance surface" on the main body of the pentaprism is in particular the surface of the pentaprism through which the incoming beams (incident beams) comprising the first and second spectral regions enter the pentaprism. A region of the entrance surface on the main body may act as an entrance aperture, and regions outside of an effective diameter of the incoming beam may be made opaque to incoming light. Preferably, incoming beams only enter the pentaprism via the entrance surface of the main body. Therefore, areas of the entrance surface of the main body and/or non-beam passing surface of the main body and the prism wedge may be coated with an opaque substance, such as a black paint, to avoid any stray or unwanted light from passing thereinto.

The "second internal reflective surface" is in particular a surface of the main body of the pentaprism, on which the split first beams that are split by the dichroic beam splitting layer arranged between the main body and prism wedge are reflected internally in the main body and directed towards the first image sensor. The "second internal reflective surface" of the main body in particular directs the split first beams perpendicular to the longitudinal axis of the pentaprism and to the image plane of the first image sensor. In particular, the second internal reflective surface is a mirror surface serving as internal reflection surface. Preferably, the second internal reflective surface is the only reflective surface of the main body with exception of the dichroic beam splitting layer.

A "first exit surface" is in particular a surface on the main body of the pentaprism that is substantially parallel to the longitudinal axis of the pentaprism and/or the shaft. The first exit surface is in particular the longest side of the pentaprism in the sectional view along the longitudinal axis. The first beams split and reflected by the second internal reflective surface of the main body in particular leave the main body through the first exit surface. In particular, the surfaces of the main body of the pentaprism are arranged such that each an angle of 90° is arranged between the incoming beams entering the main body through the entrance surface and the split and reflected first beams leaving the main body through the first exit surface and/or between the first beams reflected by the dichroic beam splitting layer and the secondly reflected beams by the second internal reflective surface leaving the main body through the first exit surface.

A "dichroic beam splitting layer" in particular is a thin layer that selectively reflects and transmits light depending on the light's wavelength. The dichroic beam splitting layer is selected such that the incident beams are split into the first beams of the first spectral region and the second beams of the second spectral region. The dichroic beam splitting layer is arranged between the main body and the prism wedge of the pentaprism. In particular, the entrance surface of the main body through which incoming beams pass through the main body is arranged substantially opposite to the surface of the main body at which, or adjacent to which, the dichroic beam splitting layer is arranged. The dichroic beam splitting layer is in particular directed at an incline to the optical axis, the longitudinal axis of the pentaprism, and/or the shaft, and towards a vertical direction perpendicular to the longitudinal axis and/or optical axis. The dichroic beam splitting layer can be a coating applied to the respective surface of the main body and/or the surface of the prism wedge, which are adjacent to each other. The adjacent surfaces of the main body and the prism wedge, with associated coating/coatings, may be adhered by optical cement. The combination of these coated surfaces and optical cement may thereby act as a dichroic filter. By the term "the dichroic beam splitting layer is arranged between the adjacent surfaces" it is understood that, in particular, the dichroic beam splitting layer and/or coating is arranged on at least one surface of both adjacent surfaces of the main body and the prism wedge.

A dichroic beam splitting layer can also be a dielectric coating or a dichroic mirror. The dichroic beam splitting layer is in particular formed as a first internal reflective and transmissive splitting layer causing the first beams to be reflected internally and directed towards the second internal reflective surface of the main body and passing the second beams towards the prism wedge. In particular, the dichroic beam splitting layer is arranged such that any kind of two spectral regions can be split and therewith separated from each other. In particular, the dichroic beam splitting layer divides the incident beams into first beams with a spectrum of 400 to 658 nm in the visible range and to second beams with a wavelength of 800 to 950 nm in the near infrared range.

A "second exit surface" is in particular a surface on the prism wedge, through which the transmitted second beams of the second spectral region leave the pentaprism. The second exit surface is in particular arranged parallel to the entrance surface of the main body. The entrance surface of the main body is in particular arranged on the distal side, and the second exit surface of the prism wedge is in particular arranged on the proximal side of the pentaprism, the entrance surface of the main body and the second exit surface of the prism wedge being opposite to each other.

A "separate optical element" is in particular any kind of optical element that modifies, controls and/or corrects a property of the first beams of the first spectral region or the second beams of the second spectral region. The separate optical element is, or two or several optical elements are, in particular arranged downstream of the pentaprism. Therewith, one separate optical element, or two or several separate optical elements, can be arranged between an outer surface of the pentaprism in the radial direction from the optical axis or along the optical axis and therewith the longitudinal axis of the pentaprism or the shaft. For a space-saving imaging device, the at least one separate optical element is arranged between the second exit surface of the prism wedge and the image sensor that is arranged with its image plane perpendicular to the optical axis and/or the longitudinal direction of the pentaprism and/or the shaft. Therefore, preferably, the one separate optical element is, or two or more separate optical elements are arranged on the proximal side of the pentaprism within the shaft of the endoscope.

The term "separate" optical element is understood such that this optical element is usually not an integral part of the pentaprism and likewise not an integral part of either of the image sensors. Therefore, the separate optical element in most embodiments is not cemented to the second exit side of the prism wedge or connected to the pentaprism or the image sensor in a direct contact, however this is not limiting, and some embodiments are envisioned where these elements may be connected and/or in direct contact. Furthermore, the term "separate" can also mean that the pentaprism and the successive optical element have different optical functions within the imaging device.

The "optical axis" is, in particular, a line along which some degree of rotational symmetry exists in an optical system. The optical axis is, in particular, an imaginary line that defines the path along which the incoming beams propagate through an objective system in front of the imaging device or enter the imaging device. Preferably, the optical axis passes through the center of curvature of each optical element within a lens system and/or objective system and/or the first entrance surface of the pentaprism of imaging device. However, the optical axis can also be bent and/or directed by a lens, an optical element, the imaging device, and/or the at least one optical element downstream of the pentaprism and/or prism wedge.

An "image sensor" has, in particular, its sensor plane in an image plane of the imaging device. The sensor plane of the respective image sensor can be arranged substantially at a distance from, and in parallel or perpendicular to the longitudinal axis of the pentaprism and/or of the shaft. In general, the terms "first image sensor" and "second image sensor" are only used for differentiation. Therefore, instead of the first image sensor, also the second image sensor can be arranged and vice versa. Preferably, the first image sensor is arranged parallel to the first exit surface arranged on the main body of the pentaprism, and the second image sensor is preferably arranged perpendicular to the longitudinal axis of the pentaprism and/or the shaft and therewith parallel to the second exit surface arranged on the prism wedge of the pentaprism. In preferred embodiments, the image size at the respective image sensor is at least 1.6 mm or above. The image planes of the first image sensor and the second image sensors can have the same size and/or properties or different sizes and/or properties. Preferably, the image sensor capturing fluorescence and/or infrared light has a smaller image size than the second image sensor capturing visible light. For example, the image sensor capturing visible light can have an image size diameter of 3.01 mm and a F-number of 6 and the image sensor capturing infrared light can have an image size diameter of 1.65 mm and a F-number of 2.8. The image sensor, in particular an electronic image sensor, may be, for example, a charge-coupled device (CCD) or a complementary metal-oxide semiconductor (CMOS). Preferably, the electronic image sensor is a high-definition (HD) image sensor having, for example, full HD resolution. In general, the electronic image sensor is configured to convert the captured image into electrical image signals and therefore image data. In particular, the electronic image sensor is arranged in the shaft and/or distal end section, e. g. the tip of the shaft, and transmits the electrical image signals from the shaft or the distal end of the shaft to its proximal end by electric transmission lines, such as wires, cables and/or a flexible printed circuit board. Preferably the electric image signals generated by the electronic image sensor are transferred from the shaft to the handle of the endoscope and/or a display system and/or a processing unit for displaying the captured images and/or superimposed imaging. Alternatively, the electrical image signals may be transmitted wirelessly, either directly from the shaft and/or the distal end section, or after being relayed to a transmitter contained in the handle. In case of a 3D-video endoscope, respectively, four image sensors are arranged in parallel, each set of two image sensors providing visible and fluorescent images from a given, distinct perspective.

With respect to "the first spectral region and the second spectral region differ at least partially from each other" it is understood that the first spectral region, and therewith a first wavelength band, and the second spectral region, and therewith a second wavelength band, do not include exactly the same wavelengths. However, the first spectral region may include completely the second spectral region or vice versa. For example, the first spectral region may comprise the wavelength range of 400 nm to 900 nm and the second spectral region may comprise the wavelength range of 700 nm to 800 nm.

"White light" (also called "visible light") is usually understood to refer to a combination wavelengths of light at from 380 nm to 750 nm, that is between the ultraviolet and infrared regions, that is, electromagnetic radiation within the portion of the spectrum perceived by a healthy human eye.

"Fluorescent light" is, in particular, an emission of light by a substance called a fluorophore that has absorbed light or other magnetic radiation. The fluorophore is usually irradiated with a specific excitation wavelength or wavelength band resulting in the emission of light with a specific emission wavelength or wavelength band. Normally, the emission wavelength is longer than the excitation wavelength. For example, in case of the commonly used fluorophore indocyanine green (ICG), the excitation wavelength range is between 600 nm and 900 nm and the emission wavelength range is between 750 nm and 950 nm in the IR spectrum. In fluorescence imaging, which is often used to optically define a tumorous region during surgery, a biological material, such a tissue in a body cavity is dyed with a fluorophore directly, or an administered substance is converted into a fluorophore by the body or a microorganism prior to imaging with an endoscope. Additionally, auto fluorescence can also be observed without previous colorization by a fluorophore or dye.

Consequently, "fluorescence light" may refer to the excitation and/or emission wavelengths or wavelength bands of a fluorophore. Radiation that causes a fluorophore to emit fluorescent light is generally referred to as "excitation light" and the resulting light emitted from the fluorophore is referred to as "emission light" or "fluorescent light". In fluorescence imaging, an optional fluorescence filter can block the excitation wavelength from reaching the detecting image sensor, and therefore the fluorescence light comprises only the light emitted by the fluorophore.

The "refractive index" of an optical medium is a dimensionless number that gives an indication of the light bending ability of the medium. The refractive index in particular determines how much the path of light is bent or refracted when entering the optical medium. For example, for the pentaprism including the prism wedge a refractive index of 1,6 or above is preferred.

In a further embodiment of the imaging device, the at least one separate optical element and/or a further separate optical element is or are a lens for demagnification of a light beam of the first spectral region and/or the second spectral region. For example, a diameter of the light beam is converged by the demagnification lens. In one example, the separate optical element can be a meniscus lens.

Even though a demagnification lens can be set in both the first split beams of the first spectral region and the second split beams of the second spectral region downstream of the pentaprism, it is especially advantageous to arrange the demagnification lens between the pentaprism and a fluorescence image sensor, by which the relative intensity (per unit area of the sensor) of the fluorescence image is increased at the expense of resolution. While, in fluorescence imaging, the resolution overall is not a limiting parameter, the increased fluorescence intensity significantly improves the signal-to-noise ratio at the fluorescence image sensor and therewith improves the overall imaging quality. By decreasing the imaging by means of a demagnification element, such as a lens, the light sensitivity of the first and/or second spectral region can be selectively increased. Consequently, a smaller shaped sensor can be used providing more light intensity in the respective spectral region. The ability to use a smaller shaped image sensor is especially advantageous for an image sensor arranged perpendicular to the longitudinal direction of the shaft due to the size limitation caused by the inner shaft diameter. Furthermore, due to the demagnification element and/or lens, aberrations during image capturing by the respective image sensor are inhibited.

For providing a variable focal length, the at least one separate optical element and/or a further separate optical element can be moveable.

Therewith, by at least one moveable optical element, the image detail on the image plane of the respective image sensor can be changed and adapted for different image sections and/or different applications. For moving the at least one separate optical element, for example a linear motor and/or a piezoelectric element can be used. Likewise, the moveable optical element can be connected by a tension wire to the operator control at the handle of an endoscope. The at least one separate optical element is in particular moveable along the longitudinal axis of the shaft.

In a further embodiment of the imaging device, the at least one separate optical element and/or a further separate optical element comprises a mirror element for redirecting the first beams of the first spectral region and/or the second beams of the second spectral region.

By arranging a separate optical element comprising a mirror element after the pentaprism, especially the respective spectral region not redirected by the pentaprism itself can be redirected by the mirror element. Therefore, for example, both image sensors can be arranged with their image planes parallel to the longitudinal axis of the pentaprism and/or the shaft. Consequently, neither of the two image sensors have to be arranged in the cross-section of the shaft perpendicular to the longitudinal axis.

A "mirror element" is in particular any optical element that reflects light and/or redirects light. The mirror element in particular comprises at least one reflective surface. The mirror element can be, for example, a planar mirror surface and/or an additional reflective prism.

For enabling fluorescence imaging and/or capturing only certain wavelengths of the respective spectral region, the at least one separate optical element and/or a further separate optical element can be a filter for blocking an excitation wavelength or for discriminating further the first spectral region and/or second spectral region.

Therefore, in fluorescence imaging, the filter as a fluorescence filter can block the excitation wavelength from reaching the respective detecting image sensor, and therefore the captured fluorescence light comprises only the light emitted by an applied fluorophore in the object field. Likewise, the wavelength range of the split first or second spectral region can be further modified by a filter, for example and edge or band filter, for discriminating certain wavelengths.

In a further embodiment of the imaging device, the dichroic beam splitting layer is arranged on a surface of the main body adjacent to a surface of the prism wedge or on a surface of the prism wedge adjacent to a surface of the main body.

By arranging the dichroic beam splitting layer on a respective surface of the main body or the prism wedge, a compact and space-saving beam splitting pentaprism is provided which is small enough to be arranged in the shaft and/or the distal end section of the shaft of an COTT endoscope.

For integrating the image capturing into the imaging device, the image device comprises the first image sensor and/or the second image sensor.

Thus, maintaining the compact design of the pentaprism and the imaging device, the first image sensor and/or the second image sensor can be arranged outside of the pentaprism and/or following the at least one optical element in a radial direction from the longitudinal axis of the shaft or perpendicular to the longitudinal axis. Consequently, the first image sensor and/or the second image sensor can likewise be arranged in the shaft and/or its distal end section. Hereby, both image sensors for capturing the first beams with the first spectral region and the second beams with the second spectral region can be arranged at a respective distance to the pentaprism and/or to the at least one optical element such that the optical pathlengths of both paths have the same value or a different value. By the same value of the optical pathlengths, an in-focus image is acquired for both the first and second beams.

In a further embodiment, the first image sensor and the second image sensor are arranged perpendicular to each other or both parallel to the optical axis of the imaging device.

Therewith, the first image sensor and the second image sensor can be arranged flexibly depending on each respective inner diameter of a shaft of an endoscope.

For enabling fluorescence imaging, a light source is assignable to the imaging device, and the light source comprises a first illumination spectral region comprising white light and a second excitation spectrum region comprising excitation light, so that a fluorophore within an illuminated scene is caused to emit fluorescent light.

Therewith, despite using just one optical path for the incoming beams before the pentaprism, by means of the beam splitting pentaprism and therewith the imaging device, white light and fluorescence images can be collected simultaneously and separately by the first image sensor dedicated to white light imaging in the visible light range and the second image sensor dedicated to fluorescence imaging. Consequently, a high overall frame rate in the overlay mode and increased sensitivity for fluorescence imaging can be obtained while maintaining the white light brightness and a high fluorescence light intensity in a chip-on-the-tip endoscope compared to conventional endoscopes shuttering between white light and fluorescence frames.

In a further embodiment of the imaging device, the second image sensor for capturing the second spectral region comprising fluorescent light has a smaller image area than the first image sensor for capturing the first spectral region comprising white light.

Due to the at least one separate optical element arranged behind the pentaprism in the proximal direction, the sensitivity of the image on the second image sensor for capturing fluorescent and/or IR light can be increased by reducing the size of the image by means of the at least one separate optical element. For example, hereby, the image size can be decreased by a factor of 45 % and therefore the image size of the second sensor can be reduced from 3.01 mm to 1.65 mm. Consequently, this results in different system F-numbers, wherein the first image sensor for visible light has an image size diameter of 3.01 mm and a F-number of 6 and the second image sensor has a reduced image size diameter of 1.65 mm and a F-number of 2.8. Hereby, the light intensity of the second sensor capturing fluorescent light and/or IR light in relation to the visible beam path was increased by a factor of 4.5.

$$\left(\frac{F\#_{VIS}}{F\#_{IR}}\right)^2 = \left(\frac{6}{2.8}\right)^2 = 4.5$$

As the fluorescent and IR image size is smaller, bigger pixels are used respectively and, therewith, a higher sensitivity and a higher resolution is achieved.

In a further aspect of the invention, the problem is solved by an objective system for an endoscope, wherein the objective system is arrangeable in a distal end section of an elongate shaft of the endoscope and at least a first image sensor for capturing images of a first spectral region and a second image sensor for capturing images of a second spectral region are arrangeable in the elongate shaft, wherein the objective system comprises an objective lens system with at least a first lens, at least a second lens, and optional further lenses in order from an objective side to receive image light and to pass the image light towards the first image sensor and the second image sensor, wherein the objective system comprises a previously described imaging device, wherein the imaging device is arranged between a most proximal lens of the objective lens system and the first image sensor and/or the second image sensor.

Thus, an objective system for an endoscope is provided with only one optical path for the two spectral regions, preferably for white light and fluorescence light imaging, wherein, after the lens system, the one optical path is split by the pentaprism into a separate first optical path for the first beams within the first spectral region and a separate second optical path for the second beams split in the second spectral region, allowing the simultaneous capture of images associated with each of the first and second spectral regions by separate dedicated image sensors.

Hereby, the objective lens system can be arranged directly at the distal tip of the shaft and/or in the distal end section of the shaft, while the imaging device can be arranged at a distance more on the proximal side or proximal section of the shaft. By this, one intermediate image or two or more intermediate images can be provided along the optical axis in the proximal direction between the objective lens system and the imaging device providing a longer focal length. Therewith, for example, the distance between the most proximal lens of the objective lens system and the respective image sensor arranged along the optical axis can be longer with i.e. 70 mm, instead of conventional of 20 mm. Consequently, it is not necessary to use a larger video objective and a consequently larger prism, which would limit the spatial arrangement of the two image sensors. By arranging the imaging device more in the proximal direction with a distance to the most proximal lens of the objective lens system, a smaller and compact design of the pentaprism and a flexible arrangement of the two image sensors can be applied.

Therewith, the function of the pentaprism is integrated into the overall system by a special optical design for video endoscopes, wherein at least one intermediate image after the most proximal lens allows a better control of the image-side focal length to reach the entire distance through the pentaprism. Additionally, the stray light behavior of the complete optical system is improved. For example, the optical design is arranged for the following optical parameters with: diameter of the optical system of 3.1 mm, focal length of -0.8 mm, F-number 4.5 to 6.0, diameter of the entrance pupil of 0.19 to 0.14, achromatized for a wavelength range of 400 to 950 nm and an image scale of 0.018.

In a further embodiment of the objective system, beams entering in and/or exiting from the pentaprism are focal and/or converging.

Consequently, an afocal beam path is not necessary in the objective system and a post-focusing, after passing through the pentaprism, likewise does not have to be applied. Despite using a focal and/or converging beam path, due to the design of the pentaprism and the subsequently arranged at least one separate optical element, commonly occurring aberrations in system known from the state of the art do not occur in the inventive objective system and an overall improved image quality is provided.

In another aspect of the invention, the problem is solved by an endoscope, in particular a medical or industrial video endoscope, with a handle, an elongate shaft, a light source, an objective system and/or an imaging processing unit and/or a display system, wherein the endoscope comprises a previously described imaging device or the objective system is a previously described objective system, so that images of the first spectral region are captured by the first image sensor and of the second spectral region are captured by the second image sensor separately in parallel and/or are displayable as superimposed images by the display system.

In particular, the endoscope can be realized as a chip-on-the-tip (COTT) endoscope, wherein the first image sensor, the second image sensor and the pentaprism are arranged in a distal end section of the elongate shaft.

The invention is further explained by the following exemplary descriptions of particular embodiments. The figures show:

Figure 1    a schematic partially three-dimensional view of an endoscope and a display system,

Figure 2    a schematic longitudinal cross-sectional view of a pentaprism,

Figure 3    a schematic longitudinal cross-sectional view of an imaging device including the pentaprism shown in Figure 2, and

Figure 4    a schematic longitudinal cross-sectional view of an objective system with a lens system and the proximally arranged imaging device.

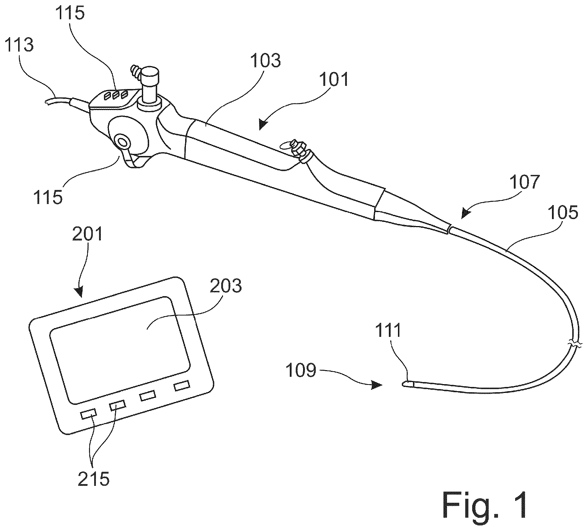

A video endoscope 101 comprises a handle 103 and an elongate shaft 105 connectable to each other at a proximal end 107 of the shaft 105. The handle 103 comprises operator controls 115 formed as buttons and a lever and is connected via a cable 113 at its proximal end to an external, control and processing unit (not shown), often referred to as a camera control unit (CCU), and/or to a display system 201 shown in Figure 1. The display system 201 includes a monitor 203 for displaying endoscopic images and operator controls 215.

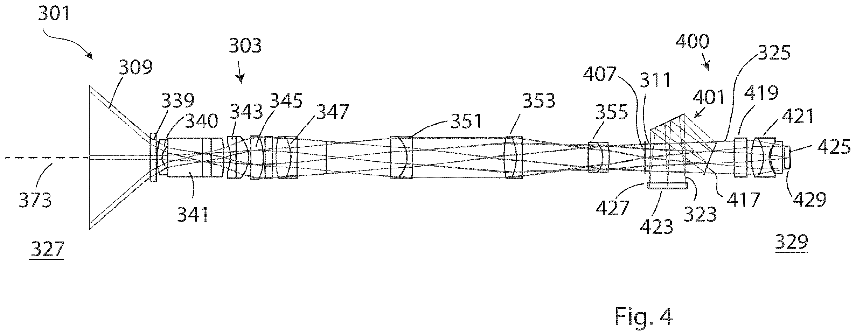

The video endoscope 101 is designed to provide video and image data from a target object space within a cavity of a body (not shown). For this, the elongate shaft 105 comprises at its distal end 109 a distal end section 111. The distal end section 111 of the elongate shaft 105 comprises an objective system 301 with an objective lens system 303 on a distal side 327 followed by an imaging device 400 on a proximal side 329 (see Figure 4).

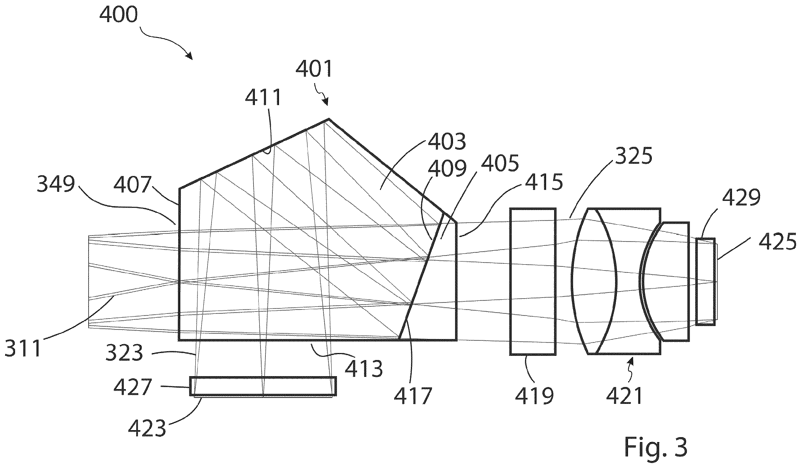

The imaging device 400 comprises a pentaprism 401 with a prism main body 403 and prism wedge 405. The prism wedge 405 is cemented to the prism main body 403. A dichroic beam splitting layer 417 is arranged between the prism main body 403 and the prism wedge 405 forming a first internal reflective and transmissive surface 409. Furthermore, the imaging device 400 comprises, in a proximal direction after the pentaprism 401 along an optical axis 373, a fluorescence filter 419 and a combined demagnification lens 421. A first image sensor 423 for capturing white light is arranged parallel to a first exit surface 413 of the pentaprism 401 and therewith parallel to the optical axis 373. On the proximal side of the demagnification lens 421, a second image sensor 425 is arranged for capturing fluorescence light (Figures 3 and 4). The fluorescence filter 419 and the demagnification lens 421 are provided as separate optical elements arranged between the pentaprism 401 and the second image sensor 425 according to the invention. In this example, they change the property of the light of the second spectral region by demagnification.

The pentaprism 401 comprises an entrance surface 407 on its prism main body 403, that is arranged perpendicular to incident beams 311 and the optical axis 373. Furthermore, the prism main body 403 comprises a second internal reflective surface 411 and a first exit surface 413 directed towards the first image sensor 423. The prism wedge 405 is arranged and cemented inclined to the prism main body 403 on the proximal side of the prism main body 403. The prism wedge 405 comprises a second exit surface 415 perpendicular to the optical axis 373.

In the objective system 301, the lens system 303 comprises, along the optical axis 373 from the distal side 327 towards the proximal side 329, a cover glass 339, a first most distal objective lens 340, followed by an attenuating and/or relaying optical element 341, a second lens 343 formed as a combined lens, a third lens 345, and a fourth lens 347 arranged in the distal end section 111 of the elongate shaft 105. At a distance to the fourth lens 347 along the optical axis 373 in a proximal direction, is a fifth lens 351 formed as a combined lens. At a distance from the fifth lens 351 is a sixth lens 353 formed as a combined lens, and at a distance therefrom is a seventh lens 355 likewise formed as a combined lens and being the most proximal lens of the lens system 303. After the seventh lens 355, as the most proximal lens, the imaging device 400 with the pentaprism 401 as described is arranged on the proximal side 329 in the distal end section 111.

The elongate shaft 105 has an inner diameter of 4 mm and the objective system 301 a diameter of 3.1 mm. The pentaprism 401 has a length along the optical axis 373 of 5 mm and the overall imaging device 400 including the pentaprism 401 of 10 mm.

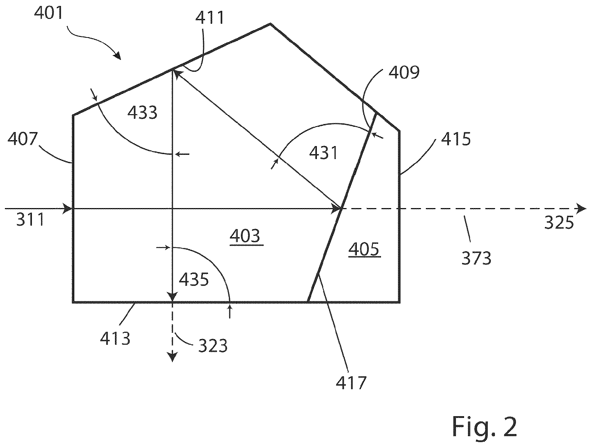

In using the video endoscope 101, object beams 309 coming from the object space at the distal side 327 are collected by the objective system 301 and conditioned by the objective lens system 303 in a single optical path with converging beams. After the last, seventh lens 355 of the lens system 303, incident beams 311 enter the pentaprism 401 through the entrance surface 407 into the prism main body 403. As both the prism main body 403 and the prism wedge 405 comprise a refraction index of 0.6, the entered incident beams 311 are split selectively by the dichroic beam splitting layer 417 into white light beams 323 and fluorescence beams 325. The reflected white light beams 323 are directed towards the second internal reflective surface 411 at a first angle 431 of 70° relative to the first internal reflective and transmissive surface 409. The split and redirected white light beams 323 are reflected by the second internal reflective surface 411 of the prism main body 403 with a second angle 433 of 65° relative to the second internal reflective surface 411 and in the direction of the first image sensor 423. Hereby, the secondly reflected white light beams 325 intersect the optical axis 373 with an angle of 90° and have a third angle 435 of 90° relative to the first exit surface 413 of the prism main body 403, wherein the beams pass the first exit surface 413, an air gap, and then, through a first sensor cover glass 427, intersect the image plane of the first image sensor 423. The fluorescence beams 325 pass the dichroic beam splitting layer 417 along the optical axis 373 and leave the prism wedge 405 through the second exit surface 415 in the proximal direction (see Figures 2, 3 and 4).

The fluorescence filter 419 arranged separately on the proximal side of the second exit surface 415 serves to absorb the excitation wavelength of the fluorescent light and only passes the emission wavelength band of the fluorophore used in the object space. Subsequently, the demagnification lens 421 converges the fluorescent beams 325 further before these beams pass an air gap, a second sensor cover glass 429, and intersect the image plane of the second image sensor 425 for capturing fluorescence light. Thereby, a smaller image size diameter of 1.65 mm is used in the second image sensor 425 capturing fluorescence light in contrast to an image size diameter of 3.01 mm of the first image sensor 423 capturing white light. Respectively, the first image sensor 423 has a F-number of 6.0 and the second image sensor 425 has a F-number of 2.8. Due to the demagnification lens 421, this reduced image size of the image captured by the second image sensor 425 is enabled, increasing the intensity of the image relative to image size on the second image sensor 425 for fluorescence light, improving the sensitivity of the image sensor 425 to the fluorescent image by increasing the signal-to-noise ratio. Simultaneously, by the demagnification lens 421, aberrations are inhibited in image capturing by the second image sensor 425.

In another example, the imaging device of the present invention can employ a separate optical element in form of a meniscus lens as for example described in the U.S. patent application number 18/498935 filed on 31.

October 2023 by the present applicant having the title "Dual Magnification Fluorescence Imaging Camera". Particularly, the meniscus lens may serve for demagnification of the second beam 325 of the second spectral region for fluorescence imaging. For example, the meniscus lens may be used instead of the lens 421 in the objective system 301 and the imaging device 400 as described above for Figures 3 and 4. The meniscus lens may be realized as a singlet as disclosed in Figure 7 of U.S. patent application number 18/498935. Alternatively, the meniscus lens may be realized as a doublet with an overall meniscus shape, particularly adapted for correcting chromatic aberrations in white light, as disclosed in Figure 8 of U.S. patent application number 18/498935. Particularly, the magnification of the fluorescence imaging channel is 0.5X to 0.9X the magnification of the white light imaging channel, i. e. the meniscus lens functions to reduce the magnification of the fluorescence imaging channel. In an advantageous example, the meniscus lens has a first radius r1 and a second radius r2, with a magnification m equal to r2/r1 being between about 0.56 and 0.88.

In a further example, the imaging device of the invention may be realized as a video camera device as disclosed in the example camera heads presented in Figures 2 and 6 of U.S. patent application number 18/498935, wherein the beamsplitter is replaced by a pentaprism as described herein. In these examples, the beamsplitter, respectively the pentaprism, is arranged on the optical axis for transmitting white light in a white light channel and for reflecting fluorescence light in a fluorescence light channel at the dichroic beam splitting layer of the pentaprism. The meniscus lens is arranged in the fluorescence channel for reducing magnification of the channel. Alternatively, the meniscus lens could be arranged as a separate optical element in the white light channel to increase the white light beam as for example disclosed in the camera head shown in Fig. 5 of U.S. patent application number 18/498935.In a further example, the imaging device of the present invention can employ a separate optical element in form of a deformable liquid lens as for example described for the camera head shown in Figure 6 the U.S. patent application number 18/498935. The liquid lens can be used for focal adjustments of the white light beam 323 and/or the fluorescence beam 325 exiting from the pentaprism by changing and adjusting the radius of curvature of the lens.

The disclosure of characteristics, alternative examples and advantages of the meniscus lens and the liquid lens as presented in the U.S. patent application number 18/498935 is fully incorporated in the specification of the present invention.

Therewith, a video endoscope 101 is provided with a compact imaging system 400 and a compact pentaprism 401 providing beam splitting into white light and fluorescence light arranged in the narrow shaft 105 allowing the simultaneously capturing of separate white light beams 323 and fluorescent beams 325 by separate first and second image sensors 423, 425 with high-quality images and a high frame rate of 60 frames per second.

Reference numerals

| 101 | video endoscope |
|---|---|
| 103 | handle |
| 105 | elongate shaft |
| 107 | proximal end of shaft |
| 109 | distal end of shaft |
| 111 | distal end section |
| 113 | cable |
| 115 | operator controls |
| 201 | display system |
| 203 | monitor |
| 215 | operators controls |
| 301 | objective system |
| 303 | lens system |
| 309 | object beams |
| 311 | incident beams |
| 323 | white light beams |
| 325 | fluorescent beams |
| 327 | distal side |
| 329 | proximal side |
| 339 | cover glass |
| 340 | first most distal objective lens |
| 341 | attenuating and/or relaying optical elements |
| 343 | second lens (combined) |
| 345 | third lens |
| 347 | fourth lens (combined) |
| 351 | fifth lens (combined) |
| 353 | sixth lens (combined) |
| 355 | seventh lens (combined) |
| 373 | optical axis |
| 400 | imaging device |
| 401 | pentaprism |
| 403 | prism main body |
| 405 | prism wedge |
| 407 | entrance surface |
| 409 | first internal reflective and transmissive surface |
| 411 | second internal reflective surface |
| 413 | first exit surface |
| 415 | second exit surface |
| 417 | dichroic beam splitting layer |
| 419 | fluorescence filter |
| 421 | demagnification lens |
| 423 | first image sensor (white light) |
| 425 | second image sensor (fluorescent light) |
| 427 | first sensor cover glass |
| 429 | second sensor cover glass |
| 431 | first angle |
| 433 | second angle |
| 435 | third angle |

**Claims**

1.  Imaging device (400) for a distal end section (111) of an endoscope (101), wherein the imaging device (400) comprises a pentaprism (401) with a main body (403) and a prism wedge (405) and an optical axis (373) and a dichroic beam splitting layer (417) as a first internal reflective and transmissive splitting layer (409), wherein an entrance surface (407), a second internal reflective surface (411) and a first exit surface (413) are arranged on the main body (403) and a second exit surface (415) is arranged on the prism wedge (405), , and at least a first image sensor (423) for capturing light of a first spectral region and a second image sensor (425) for capturing light of a second spectral region are assignable to the imaging device (400),

    wherein the first spectral region and the second spectral region differ at least partially from each other, and
    the dichroic beam splitting layer (417) is arranged between the main body (403) and the prism wedge (405) of the pentaprism (401), so that incident beams (311) comprising the first and second spectral regions are split by the dichroic beam splitting layer (417) into a first beam (323) of the first spectral region and a second beam (325) of the second spectral region,
    **characterized in that**
    at least one separate optical element (419, 421) is arranged between the pentaprism (401) and the first image sensor (423) and/or the second image sensor (425) for changing a property of the light of the first spectral region and/or the second spectral region, so that two separate images are capturable by the first image sensor (423) and the second image sensor (425) for superimposed imaging of the light of the first spectral region and the second spectral region.

2.  Imaging device (400) according to claim 1, **characterized in that** the at least one separate optical element and/or a further separate optical element is or are a meniscus lens.

3.  Imaging device (400) according to claim 1 or 2, **characterized in that** the at least one separate optical element and/or a further separate optical element (419, 421) is or are a lens (421) for demagnification of the first beam (323) of the first spectral region and/or the second beam (325) of the second spectral region.

4.  Imaging device (400) according to one of the previous claims, **characterized in that** the at least one separate optical element and/or a further separate optical element (419, 421) is or are movable.

5. Imaging device (400) according to one of the previous claims, **characterized in that** the at least one separate optical element and/or a further separate optical element (419, 421) comprises a mirror element for redirecting the first beam (323) of the first spectral region and/or the second beam (325) of the second spectral region.

6. Imaging device (400) according to one of the previous claims, **characterized in that** the at least one separate optical element and/or a further separate optical element (419, 421) is or are a filter (419) for blocking an excitation wavelength or for discriminating further the first spectral region and/or second spectral region.

7. Imaging device (400) according to one of the previous claims, **characterized in that** the dichroic beam splitting layer (417) is arranged on a surface of the main body (403) adjacent to a surface of the prism wedge (405) or on a surface of the prism wedge (405) adjacent to a surface of the main body (403).

8. Imaging device (400) according to one of the previous claims, **characterized in that** the imaging device (401) comprises the first image sensor (423) and/or the second image sensor (425).

9. Imaging device (400) according to one of the previous claims, **characterized in that** the first image sensor (423) and the second image sensor (425) are arranged perpendicular to each other or both parallel to the optical axis (373) of the imaging device (400).

10. Imaging device (400) according to one of the previous claims, **characterized in that** a light source is assignable to the imaging device (400), and the light source comprises a first illumination spectral region comprising white light and a second excitation spectral region comprising excitation light, so that a fluorophore within an illuminated scene is caused to emit fluorescent light.

11. Imaging device (400) according to claim 10, **characterized in that** the second image sensor (425) for capturing the second spectral region comprising fluorescent light has a smaller image area than the first image sensor (423) for capturing the first spectral region comprising white light.

12. Objective system (301) for an endoscope (101), wherein the objective system (301) is arrangeable in a distal end section (111) of an elongate shaft (105) of the endoscope (101) and at least a first image sensor (423) for capturing images of a first spectral region and a second image sensor (425) for capturing images of a second spectral region are arrangeable in the elongate shaft (105), wherein the objective system (301) comprises an objective lens system (303) with at least a first lens (340), at least a second lens (343) and optional further lenses (345, 347, 349, 351, 353, 355) in order from an objective side to receive image light and to pass the image light towards the first image sensor (423) and the second image sensor (425), **characterized in that** the objective system (301) comprises an imaging device (400) according to one of the claims 1 to 10, wherein the imaging device (400) is arranged between a most proximal lens (355) of the objective lens system (301) and the first image sensor and/or the second image sensor (423, 425).

13. Objective system (301) according to claim 12, **characterized in that** beams entering in and/or exiting from the pentaprism (401) are focal and/or converging.

14. Endoscope (101), in particular a medical or industrial video endoscope, with a handle (103), an elongate shaft (105), a light source, an objective system and/or an image processing unit and/or a display system (201), **characterized in that** the endoscope (101) comprises an imaging device (400) according to one of the claims 1 to 10 or the objective system is an objective system (301) according to claim 11 or 12, so that images of the first spectral region are captured by the first image sensor (423) and of the second spectral region are captured by the second image sensor (425) separately in parallel and/or are displayable as superimposed images by the display system (201).

15. Endoscope according to claim 13, **characterized in that** the endoscope is realized as a chip-on-the-tip (COTT) endoscope wherein the first image sensor (423), the second image sensor (425) and the pentaprism (401) are arranged in a distal end section (111) of the elongate shaft (105).

Fig. 1

Fig. 2

Fig. 3

EP 4 553 560 A1

Fig. 4

| | Europäisches Patentamt European Patent Office Office européen des brevets | **EUROPEAN SEARCH REPORT** | **Application Number** EP 24 20 8616 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/265490 A1 (DUCKETT III GEORGE E [US]) 29 August 2019 (2019-08-29) | 1,4-14 | INV. G02B23/24 |
| Y | * paragraphs [0028] - [0034]; figures 2-6 * | 15 | G02B27/10 G02B27/14 |
| | - - - - - | | |
| Y | DE 10 2010 028590 A1 (WINTER & IBE OLYMPUS [DE]) 10 November 2011 (2011-11-10) * the whole document * | 15 | |
| | - - - - - | | |
| A | US 2012/105594 A1 (YOU JANG-WOO [KR] ET AL) 3 May 2012 (2012-05-03) * the whole document * | 1-15 | |
| | - - - - - | | |
| A | US 2020/088579 A1 (BALAS KONSTANTINOS [GR]) 19 March 2020 (2020-03-19) * the whole document * | 1-15 | |
| | - - - - - | | |

**TECHNICAL FIELDS SEARCHED (IPC)**

G02B

The present search report has been drawn up for all claims

| Place of search | Date of completion of the search | Examiner |
|---|---|---|
| Munich | 28 March 2025 | Wolf, Steffen |

EPO FORM 1503 03.82 (P04C01)

## ANNEX TO THE EUROPEAN SEARCH REPORT
## ON EUROPEAN PATENT APPLICATION NO.

EP 24 20 8616

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2025

| Patent document cited in search report | Publication date | Patent family member(s) | | Publication date |
|---|---|---|---|---|
| US 2019265490 A1 | 29-08-2019 | NONE | | |
| DE 102010028590 A1 | 10-11-2011 | NONE | | |
| US 2012105594 A1 | 03-05-2012 | EP | 2458424 A1 | 30-05-2012 |
| | | KR | 20120045462 A | 09-05-2012 |
| | | US | 2012105594 A1 | 03-05-2012 |
| US 2020088579 A1 | 19-03-2020 | CN | 112789495 A | 11-05-2021 |
| | | EP | 3830551 A1 | 09-06-2021 |
| | | JP | 7087198 B2 | 20-06-2022 |
| | | JP | 2021527228 A | 11-10-2021 |
| | | KR | 20210043595 A | 21-04-2021 |
| | | US | 2020088579 A1 | 19-03-2020 |
| | | WO | 2020025987 A1 | 06-02-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200088579 A1
- US 20140225992 A1
- US 49893523
- US 498935
- US 498935 A